(19) Europäisches Patentamt / European Patent Office / Office européen des brevets

(11) **EP 2 204 162 B1**

(12) **FASCICULE DE BREVET EUROPEEN**

(45) Date de publication et mention de la délivrance du brevet:
**31.08.2016 Bulletin 2016/35**

(51) Int Cl.:
*A61K 8/60* *(2006.01)*     *A61Q 5/12* *(2006.01)*
*A61Q 7/00* *(2006.01)*     *A61Q 17/04* *(2006.01)*
*A61Q 19/08* *(2006.01)*

(21) Numéro de dépôt: **09181013.5**

(22) Date de dépôt: **30.12.2009**

(54) **Utisilisation de monosaccharides et composition**

Verwendung von Monosacchariden und Zusammensetzung

Use of monosaccharides and composition

(84) Etats contractants désignés:
**AT BE BG CH CY CZ DE DK EE ES FI FR GB GR HR HU IE IS IT LI LT LU LV MC MK MT NL NO PL PT RO SE SI SK SM TR**

(30) Priorité: **30.12.2008 FR 0859147**
             **15.01.2009 US 144774 P**

(43) Date de publication de la demande:
**07.07.2010 Bulletin 2010/27**

(73) Titulaire: **L'Oréal**
**75008 Paris (FR)**

(72) Inventeurs:
• **Simonnet, Jean-Thierry**
**94230, Cachan (FR)**
• **Laboureau, Juilen**
**92130, Issy les Moulineaux (FR)**
• **Portes, Pascal**
**94130, Nogent sur Marne (FR)**
• **Boulle, Christophe**
**75005, Paris (FR)**

(74) Mandataire: **Tezier Herman, Béatrice et al**
**Cabinet Becker & Associés**
**25, rue Louis le Grand**
**75002 Paris (FR)**

(56) Documents cités:
| | |
|---|---|
| WO-A-2007/101493 | WO-A-2008/148966 |
| WO-A2-2007/007095 | DE-A1- 19 503 423 |
| DE-A1- 19 704 693 | FR-A- 2 756 735 |
| FR-A- 2 768 623 | FR-A- 2 773 324 |
| FR-A- 2 876 283 | FR-A1- 2 609 397 |
| FR-A1- 2 853 539 | FR-A1- 2 900 574 |
| FR-A1- 2 912 313 | US-A1- 2007 025 933 |

**Description**

La présente invention concerne l'utilisation, notamment cosmétique et/ou dermatologique, d'au moins un monosaccharide choisi parmi le mannose, le rhamnose et leur mélange pour diminuer ou prévenir les signes du vieillissement de la peau ou de ses phanères. La présente invention concerne également une composition cosmétique comprenant dans un milieu physiologiquement acceptable, au moins un monosaccharide choisi parmi le mannose, le rhamnose et leur mélange, ainsi qu'un dispositif le contenant.

La peau humaine est constituée de deux couches principales qui sont le derme et l'épiderme qui recouvre le derme de manière superficielle. L'épiderme humain naturel est composé principalement de trois types de cellules qui sont les kératinocytes, très majoritaires, les mélanocytes et les cellules de Langerhans. Chacun de ces types cellulaires contribue par ses fonctions propres au rôle essentiel joué dans l'organisme par la peau, notamment le rôle de protection de l'organisme des agressions extérieures (climat, rayons ultraviolets, tabac...), appelé « fonction barrière ».

L'épiderme est un épithélium pavimenteux stratifié kératinisé constitué à 90% de kératinocytes. La différenciation progressive des cellules de la membrane basale, qui sépare le derme de l'épiderme, vers la surface de l'épiderme comprend notamment la différenciation des kératinocytes qui migrent vers la surface de la peau où ils desquament.

Le vieillissement de l'épiderme se manifeste principalement par la réduction de son épaisseur. L'atrophie de l'épiderme est la conséquence du ralentissement de la prolifération des kératinocytes et de l'accumulation de kératinocytes sénescents. La couche cornée devient terne.

Le derme fournit à l'épiderme un support solide. C'est également son élément nourricier. Il est principalement constitué de fibroblastes et d'une matrice extracellulaire composée majoritairement de collagène, d'élastine et d'une substance, dite substance fondamentale. Ces composants sont synthétisés par les fibroblastes. La cohésion entre l'épiderme et le derme est assurée par la jonction dermo-épidermique. C'est une région complexe d'une épaisseur de 100 nm environ qui comprend le pole basal des kératinocytes basaux, la membrane épidermique et la zone sous-basale du derme superficiel.

Les collagènes sont les protéines majoritaires des matrices extracellulaires de la peau. A ce jour, 20 types de collagènes sont identifiés et notés de I à XX. Les collagènes majoritairement présents dans l'ensemble du derme sont les collagènes de type I et III qui forment la matrice extracellulaire de l'ensemble du derme (ce sont ces collagènes qui constituent 70-80 % du poids sec du derme). Par ailleurs, les collagènes ne sont pas tous synthétisés par les mêmes types cellulaires, les collagènes de type I et III sont essentiellement produits par les fibroblastes dermiques alors que le collagène de type VII est produit par deux catégories de cellules, les kératinocytes et les fibroblastes. La régulation de leur expression diffère d'un collagène à un autre, par exemple, les collagènes I et VII ne sont pas régulés de la même manière par certaines cytokines, en effet, le TNF alpha et la leukoréguline stimulent le collagène VII et régulent négativement le collagène I. Enfin, toutes les molécules de collagène sont des variantes d'un précurseur commun qui est la chaîne alpha du procollagène.

Avec le vieillissement, le collagène s'amincit et des rides apparaissent à la surface de la peau. Le vieillissement cutané est un mécanisme génétiquement programmé.

Par ailleurs, certains facteurs d'environnement comme le tabagisme et l'exposition au rayonnement du soleil l'accélèrent. La peau a ainsi un aspect beaucoup plus âgé sur les zones exposées au soleil comme le dos des mains ou le visage. Ainsi, ces autres facteurs ont également un impact négatif sur le collagène naturel de la peau.

En conséquence, compte tenu du rôle important du collagène au niveau de l'intégrité de la peau et de sa résistance aux agressions externes de type mécaniques, la stimulation de synthèse de ces collagènes, et en particulier du collagène de type I, apparaît comme un moyen efficace pour pallier les signes du vieillissement cutané. Au cours du vieillissement chronologique, l'épiderme subit également de nombreuses modifications et dégradations qui se traduisent, avec l'âge, par une altération du microrelief associée à une altération de la fonction barrière de la peau, une apparition de rides et ridules, une altération des propriétés mécaniques de la peau, notamment un manque d'élasticité de la peau, et une perte d'éclat du teint.

On comprend donc l'importance de pouvoir disposer de produits dont les effets visent à régénérer le tissu de la peau via une augmentation de la prolifération des kératinocytes, une stimulation de la prolifération et/ou du métabolisme des fibroblastes, et notamment une stimulation de la synthèse des collagènes.

Il est connu de la littérature l'utilisation d'agents tels que le rétinol qui favorisent la prolifération des kératinocytes et permettent de stimuler le renouvellement de l'épiderme, maintenir et/ou augmenter l'épaisseur de l'épiderme, on parle alors d'effet biologique direct. Cependant, le rétinol présente un certain nombre de désavantages lors de son utilisation dans une composition cosmétique. En effet, il a une faible stabilité vis-à-vis de l'oxydation et génère des effets secondaires indésirables pour les consommateurs, tels que notamment l'irritation de la peau. Il existe donc un besoin de trouver d'autres composés présentant un effet biologique direct qui soient facilement accessibles et qui présentent une efficacité acceptable pour une utilisation optimale en cosmétique.

La demanderesse a démontré, de façon surprenante et inattendue, que les monosaccharides choisis parmi le mannose, le rhamnose et leur mélange présentent l'activité recherchée dans ce contexte, et dans le cadre des contraintes imposées mentionnées au paragraphe précédent.

Ainsi, les inventeurs ont constaté que le mannose et/ou le rhamnose sont capables d'augmenter le nombre de kératinocytes et/ou de fibroblastes, de stimuler le métabolisme des fibroblastes, en stimulant la synthèse des collagènes, en particulier la synthèse de procollagène de type 1; et ainsi de contrer les signes du vieillissement cutané, et en particulier l'atrophie épidermique et/ou dermique liée au vieillissement.

L'utilisation de ces monosaccharides est restée jusqu'alors inconnue pour les effets biologiques directs exposés plus haut. La demande WO 2007/128939 mentionne une activité anti-âge obtenue par un effet biomécanique d'un agent tenseur en association avec des composés saccharidiques, ce qui permet d'augmenter l'expression des mécanorécepteurs de cellules de la peau. Cette augmentation de l'expression des mécanorécepteurs est décrite comme augmentant la sensibilisation des cellules de la peau à répondre aux effets des tenseurs. De même, la demande française FR2900572 mentionne un procédé de soin cosmétique de la peau impliquant l'utilisation conjointe d'une composition comprenant un composé saccharidique permettant d'augmenter l'expression des mécanorécepteurs de cellules de la peau et d'un dispositif destiné à appliquer des contraintes mécaniques sur la peau, ce qui permet d'augmenter l'efficacité des contraintes mécaniques grâce à l'augmentation du nombre de mécanorécepteurs de cellules de la peau.

La demande de brevet US 2007/0025933 décrit une composition comprenant une base photoprotectrice, constituée de deux types de composants, et éventuellement un mélange de composants additionnels, tels que notamment des monosaccharides (comme le mannose, fructose et glucose) et des acides du cycle de Krebs ou leurs dérivés (comme l'acide citrique, malique, fumarique) pour stabiliser ladite composition. Aucune activité propre aux monosaccharides sur la peau n'est mentionnée.

La demande WO 2005/063194 décrit une base galénique à très haute tolérance comprenant notamment du mannose ou du rhamnose. Il est spécifié qu'une telle base galénique ne peut fonctionner qu'en association avec un actif dont la base galénique est seulement le véhicule. Les bases galéniques dermiques et/ou cosmétiques divulguées reposent essentiellement sur la présence des deux polyols que sont le mannitol et le xylitol.

Le mannose est un hexose épimère en C2 du glucose. Le rhamnose (ou 6 déoxy mannose) constitue formellement le produit de désoxygénation du mannose en C6. Les monosaccharides selon l'invention sont sous la forme D ou L du mannose et/ou du rhamnose ou leur mélange, chaque forme pouvant elle-même être l'anomère alpha et/ou béta. Les formes préférées selon l'invention sont le D-mannose ou le L-rhamnose.

Le D-mannose est présent dans les végétaux en particulier certains fruits dont les airelles (canneberges), ou le bois dur (hêtre, bouleau). Le rhamnose est trouvé dans la nature sous forme L. Le D-mannose, ainsi que le L-rhamnose, sont commercialisés, par exemple, par la société Danisco Sweeteners® ou bien la société Symrise.

Dans la présente invention, le monosaccharide est plus spécifiquement sous forme de monomère.

La présente invention concerne donc l'utilisation, notamment cosmétique ou dermatologique, d'au moins un monosaccharide choisi parmi le mannose, le rhamnose et leur mélange pour diminuer ou prévenir les signes du vieillissement de la peau ou de ses phanères, pour augmenter la prolifération des kératinocytes et/ou des fibroblastes et/ou pour stimuler la synthèse de collagène.

Selon un aspect particulier, l'invention porte sur l'utilisation, notamment cosmétique ou dermatologique, d'au moins un monosaccharide choisi parmi le mannose, le rhamnose et leur mélange pour stimuler la régénération de la peau, en particulier de l'épiderme et/ou du derme, grâce à un meilleur renouvellement cellulaire de la peau, en particulier de l'épiderme et/ou du derme.

Le monosaccharide selon l'invention permet de stimuler la régénération des cellules de l'épiderme et du derme, au niveau de la peau ou des phanères, en particulier les kératinocytes et les fibroblastes, par augmentation de leur prolifération. On dispose de la sorte d'une méthode, notamment cosmétique, efficace notamment pour lutter contre les signes du vieillissement, plus spécifiquement du chronovieillissement. Les signes du chronovieillissement correspondent aux dégradations internes de la peau dues au vieillissement intrinsèque des individus. L'agent actif, à savoir le monosaccharide tel que défini ci-dessus, permet de maintenir et/ou renforcer l'épiderme et/ou le derme, maintenir l'intégrité et/ou l'épaisseur des différentes couches de la peau, et en particulier l'épiderme et/ou le derme. Il permet également de maintenir l'élasticité et la tonicité de la peau.

Un autre aspect de l'invention concerne l'utilisation cosmétique et/ou dermatologique d'au moins un monosaccharide choisi parmi le mannose, le rhamnose et leur mélange pour traiter, de manière préventive ou curative, les signes du vieillissement de la peau, en particulier du corps ou du visage, en particulier les signes chronobiologiques du vieillissement de la peau, et notamment le vieillissement généré par une diminution de l'élasticité de la peau et/ou par une dégradation du collagène dans la structure des tissus.

Un autre objet de l'invention est l'utilisation d'au moins un monosaccharide choisi parmi le mannose, le rhamnose et leur mélange pour traiter, de manière préventive ou curative, ou pour diminuer ou prévenir les rides et/ou ridules, la peau flétrie, le manque d'élasticité et/ou de tonus de la peau, l'amincissement du derme, la dégradation des fibres de collagène, la peau molle, la peau amincie, ou toute dégradation interne de la peau.

Un autre objet de l'invention est l'utilisation d'au moins un monosaccharide choisi parmi le mannose, le rhamnose et leur mélange pour maintenir et/ou améliorer la fonction barrière de la peau.

Un autre objet de l'invention est l'utilisation d'au moins un monosaccharide choisi parmi le mannose, le rhamnose et leur mélange pour maintenir l'éclat de la peau et/ou donner une peau éclatante.

L'invention porte également sur l'utilisation, notamment cosmétique, d'au moins un monosaccharide choisi parmi le mannose, le rhamnose et leur mélange pour améliorer et/ou diminuer le micro-relief de la peau ou encore pour maintenir et/ou améliorer la fonction barrière de la peau.

L'utilisation du monosaccharide conforme à l'invention permet de lutter efficacement contre les signes du vieillissement de la peau du corps ou du visage. Le monosaccharide selon l'invention peut ainsi traiter, de manière préventive ou curative, les rides et/ou ridules. Le monosaccharide selon l'invention peut également retarder ou prévenir l'apparition des rides et/ou ridules.

La présente invention porte de manière plus spécifique sur l'utilisation cosmétique d'au moins un monosaccharide choisi parmi le mannose, le rhamnose et leur mélange pour traiter, de manière préventive ou curative, les rides et/ou ridules, et/ou toute dégradation interne de la peau.

La présente invention concerne également l'utilisation cosmétique d'au moins un monosaccharide choisi parmi le mannose, le rhamnose et leur mélange, pour maintenir et/ou renforcer l'épiderme et/ou le derme, pour maintenir l'intégrité et/ou l'épaisseur des différentes couches de l'épiderme et du derme.

Le monosaccharide selon l'invention peut être administré par voie orale, topique sur la peau ou ses phanères ou par injection cutanée.

Plus spécifiquement, le mannose, le rhamnose ou leur mélange sont utilisés, à titre d'agent actif dans une composition cosmétique ou dermatologique.

La présente invention porte aussi sur l'utilisation d'au moins un monosaccharide choisi parmi le mannose, le rhamnose et leur mélange, administré par voie orale, topique ou par injection cutanée, notamment pour le soin de la peau et/ou du cuir chevelu.

Une composition cosmétique pour soin capillaire conforme à l'invention comprenant au moins un monosaccharide choisi parmi le mannose, le rhamnose et leur mélange permet en particulier la stimulation de la pousse du cheveu, la lutte contre la chute des cheveux, le ralentissement de sa chute ou le renforcement de l'éclat du cheveu.

La présente invention concerne d'autre part une composition cosmétique et/ou dermatologique comprenant, dans un milieu physiologiquement acceptable, au moins un monosaccharide choisi parmi le mannose, le rhamnose et leur mélange, ladite composition ne comprenant pas d'agent tenseur et ne comprenant pas l'association de xylitol et de mannitol.

Ainsi, la composition selon l'invention peut être destinée aux utilisations telles que précédemment décrites.

Le monosaccharide selon l'invention est plus particulièrement présent dans la composition selon l'invention à titre d'agent (ou ingrédient) actif, en particulier de seul agent actif.

Par agent actif ou ingrédient actif, on entend plus spécifiquement selon l'invention, un composé qui, lorsqu'il est administré à un sujet, en particulier un sujet humain, joue un rôle biologique direct sur l'organisme, en particulier sur la peau ou ses phanères, en particulier sans améliorer l'effet biologique ou mécanique d'un autre composé présent dans la composition selon l'invention.

Plus particulièrement, la composition selon l'invention ne comprend pas de monosaccharide additionnel.

De manière générale, on entend par « agent tenseur » tous composés solubles ou dispersibles dans l'eau à une température allant de 25°C à 50°C à la concentration de 7% en poids dans l'eau ou à la concentration maximale à laquelle ils forment un milieu d'apparence homogène et produisant à cette concentration de 7% ou à cette concentration maximale dans l'eau une rétractation de plus de 15 % dans le test décrit ci-après.

La concentration maximale à laquelle ils forment un milieu d'apparence homogène est déterminée à $\pm$ 20% près et de préférence à $\pm$ 5% près.

On entend par "milieu d'apparence homogène" un milieu ne présentant pas d'agrégats visibles à l'oeil nu.

Pour la détermination de ladite concentration maximale, l'agent tenseur est ajouté progressivement dans l'eau sous agitation à la défloculeuse à une température allant de 25°C à 50°C, puis le mélange est maintenu sous agitation pendant une heure. On observe ensuite après 24 heures si le mélange ainsi préparé est d'apparence homogène (absence d'agrégats visibles à l'oeil nu).

L'effet tenseur peut être caractérisé par un test *in vitro* de rétractation.

On prépare préalablement et tel que décrit précédemment un mélange homogène de l'agent tenseur dans l'eau, à la concentration de 7% en poids ou à la concentration maximale définie précédemment.

30$\mu$l du mélange homogène est déposé sur une éprouvette rectangulaire (10x40mm, donc présentant une largeur initiale $L_0$ de 10 mm) d'élastomère ayant un module d'élasticité de 20MPa et une épaisseur de 100$\mu$m.

Après 3h de séchage à 22$\pm$3°C et 40$\pm$10% d'humidité relative HR, l'éprouvette d'élastomère présente une largeur rétractable, notée $L_{3h}$ due à la tension exercée par l'agent tenseur déposé.

L'effet tenseur (ET) dudit agent est alors qualifié de la façon suivante :

$$\text{'ET'} = (L_0 - L_{3h} / L_0) \times 100 \text{ en \%}$$

avec $L_0$ = largeur initiale 10mm

et $L_{3h}$ = largeur après 3h de séchage

L'agent tenseur peut être choisi parmi :

a) les protéines végétales ou animales et leurs hydrolysats ;
b) les polysaccharides d'origine naturelle ;
c) les silicates mixtes ;
d) les particules colloïdales de charges inorganiques ;
e) les polymères synthétiques ;

et les mélanges de ceux-ci.

L'homme du métier saura choisir, dans les catégories chimiques listées ci-dessus, les matériaux répondant au test tel que décrit précédemment.

La présente invention concerne aussi une composition comprenant du mannose et/ou du rhamnose et adaptée à une administration topique se présentant avantageusement sous forme d'une crème, d'un gel, d'une lotion, d'un lait, d'une huile, d'un onguent, d'une cire, d'une mousse, d'une pâte, d'un sérum, d'une pommade ou d'un shampooing.

Le monosaccharide selon l'invention, à savoir le mannose et/ou le rhamnose, et en particulier le rhamnose, est également avantageusement utilisé pour le traitement de (ou pour la préparation de compositions pharmaceutiques destinées au traitement de) désordres cutanés liés à une prolifération des cellules de l'épiderme ou du derme, en particulier les kératinocytes ou les fibroblastes, anormalement faible. Le monosaccharide selon l'invention peut être utilisé pour le traitement de (ou pour la préparation de compositions pharmaceutiques destinées au traitement de) désordres cutanés liés à une diminution du taux de collagène. En particulier, lesdits désordres sont tels que ceux identifiés ci-dessus. La composition peut être destinée à diminuer ou prévenir les signes du vieillissement de la peau ou de ses phanères, en particulier en augmentant la prolifération des kératinocytes, des fibroblastes et/ou en stimulant la synthèse de collagène, et en particulier du collagène de type I. De préférence, la composition pharmaceutique est une composition dermatologique.

La présente invention a également pour objet une méthode de traitement, en particulier cosmétique ou thérapeutique, pour diminuer ou prévenir les signes du vieillissement de la peau ou de ses phanères, par administration à un sujet, de préférence un être humain, d'une quantité efficace d'au moins un monosaccharide tel que défini précédemment ou d'une composition selon l'invention.

La quantité de monosaccharide à mettre en oeuvre selon l'invention dépend de l'effet cosmétique ou thérapeutique recherché, et peut donc varier dans une large mesure.

L'homme de l'art peut aisément, sur la base de ses connaissances générales, déterminer les quantités appropriées.

Une composition selon l'invention peut comprendre au moins un monosaccharide tel que défini ci-dessus en une quantité comprise entre 0,001 % et 30 % en poids par rapport au poids total de la composition, et en particulier entre 0,1% et 10 % en poids, et plus particulièrement entre 0,5 % et 6 % en poids par rapport au poids total de la composition.

La composition selon l'invention comprend au moins un monosaccharide tel que défini précédemment en association avec un milieu physiologiquement acceptable, en particulier un milieu cosmétiquement ou pharmaceutiquement acceptable.

D'une manière générale, ce milieu peut être anhydre ou aqueux. Il peut ainsi comprendre une phase aqueuse et/ou une phase grasse.

Le milieu physiologiquement acceptable dans lequel les composés selon l'invention peuvent être employés, ainsi que ses constituants, leur quantité, la forme galénique de la composition, son mode de préparation, et son mode d'administration peuvent être choisis par l'homme du métier sur la base de ses connaissances générales en fonction du type de composition recherchée.

Lorsque la composition est une composition destinée à une administration topique, elle peut se présenter avantageusement sous la forme de solutions aqueuses, hydroalcooliques, d'émulsions huile-dans-eau (H/E) ou eau-dans-huile (E/H) ou multiple (triple : E/H/E ou H/E/H), des nanoémulsions, en particulier des nanoémulsions H/E, dont la taille des gouttes est inférieure à 100nm, de gels aqueux, ou de dispersions d'une phase grasse dans une phase aqueuse à l'aide de sphérules, ces sphérules pouvant être des nanoparticules polymériques telles que les nanosphères et les nanocapsules ou des vésicules lipidiques de type ionique et/ou non ionique (liposomes, niosomes, oléosomes).

Ces compositions sont préparées selon les méthodes usuelles.

En outre, les compositions utilisables selon l'invention peuvent être plus ou moins fluides et avoir l'aspect d'une crème blanche ou colorée, d'une pommade, d'un lait, d'une lotion, d'un sérum, d'une pâte ou d'une mousse. Elles peuvent être éventuellement appliquées sur la peau sous forme d'aérosol. Elles peuvent aussi se présenter sous forme solide, et par exemple sous forme de stick.

Pour une application locale sur les cheveux ou le cuir chevelu, la composition peut être sous forme de solutions aqueuses, alcooliques ou hydroalcooliques; sous forme de crèmes, de gels, d'émulsions, de mousses; sous forme de compositions pour aérosol comprenant également un agent propulseur sous pression.

Lorsque la composition se présente sous forme aqueuse, notamment sous forme de dispersion, d'émulsion ou de solution aqueuse, elle peut comprendre une phase aqueuse, qui peut comprendre de l'eau, une eau florale et/ou une eau minérale.

Lorsque la composition est une émulsion, la proportion de la phase grasse peut varier d'environ 5 % à 80 % en poids, et de préférence d'environ 2 % à 50 % en poids par rapport au poids total de la composition. Les huiles, les cires, les émulsionnants et les co-émulsionnants utilisés dans la composition sous forme d'émulsion sont choisis parmi ceux classiquement utilisés dans le domaine cosmétique. L'émulsionnant et le co-émulsionnant sont présents, dans la composition, en une proportion allant de 0,3 % à 30 % en poids, et de préférence de 0,5 % à 20 % en poids par rapport au poids total de la composition. L'émulsion peut, en outre, contenir des vésicules lipidiques.

Lorsque la composition est une solution ou un gel huileux, la phase grasse peut représenter plus de 90 % du poids total de la composition.

La phase huileuse peut comprendre aussi tout additif habituel liposoluble ou lipodispersible comme indiqué ci-après.

Elle peut notamment comprendre des corps gras tels que des cires, des composés pâteux, des alcools gras, des acides gras. La phase huileuse contient au moins une huile, plus particulièrement au moins une huile cosmétique. On entend par « huile » un corps gras liquide à la température ambiante (25°C).

Comme huiles utilisables dans la composition de l'invention, on peut citer par exemple :

- les huiles hydrocarbonées d'origine animale, telles que le perhydrosqualène;
- les huiles hydrocarbonées d'origine végétale, telles que les triglycérides liquides d'acides gras comportant de 4 à 10 atomes de carbone comme les triglycérides des acides heptanoïque ou octanoïque ou encore, par exemple, les huiles de tournesol, de maïs, de soja, de courge, de pépins de raisin, de sésame, de noisette, d'abricot, de macadamia, d'arara, de coriandre, de ricin, d'avocat, les triglycérides des acides caprylique/caprique comme ceux commercialisés par la société Stearineries Dubois ou ceux commercialisés sous les dénominations Miglyol 810, 812 et 818 par la société Dynamit Nobel, l'huile de jojoba, l'huile de beurre de karité, le caprylyl glycol ;
- les esters et les éthers de synthèse, notamment d'acides gras, comme les huiles de formules R1COOR2 et R1OR2 dans lesquelles R1 représente le reste d'un acide gras ou d'un alcool gras comportant de 8 à 29 atomes de carbone, et R2 représente une chaîne hydrocarbonée, ramifiée ou non, contenant de 3 à 30 atomes de carbone, comme par exemple l'huile de Purcellin, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéaryl-malate, le citrate de triisocétyle, les heptanoates, octanoates, décanoates d'alcools gras ; les esters de polyol, comme le dioctanoate de propylène glycol, le diheptanoate de néopentylglycol et le diisononanoate de diéthylèneglycol ; et les esters du pentaérythritol comme le tétraisostéarate de pentaérythrytyle, l'isopropyl lauroyl sarcosinate, notamment vendu sous le nom commercial Eldew SL 205 de la société Ajinomoto ;

- les hydrocarbures linéaires ou ramifiés, d'origine minérale ou synthétique, tels que les huiles de paraffine, volatiles ou non, et leurs dérivés, la vaseline, les polydécènes, l'isohexadécane, l'isododécane, le polyisobutène hydrogéné tel que l'huile de Parléam, le mélange de n-undécane (C11) et de n-tridécane (C13) commercialisé sous la référence de CETIOL UT par la Société Cognis ;
- les huiles fluorées partiellement hydrocarbonées et/ou siliconées comme celles décrites dans le document JP-A-2-295912 ;
- les huiles de silicone comme les polyméthylsiloxanes (PDMS) volatiles ou non à chaîne siliconée linéaire ou cyclique, liquides ou pâteux à température ambiante, notamment les huiles de silicone volatiles, en particulier cyclopolydiméthylsiloxanes (cyclométhicones) telles que le cyclohexadiméthylsiloxane et le cyclopentadiméthylsiloxane ; lespolydiméthyl-siloxanes comportant des groupements alkyle, alcoxy ou phényle, pendants ou en bout de chaîne siliconée, groupements ayant de 2 à 24 atomes de carbone ; les silicones phénylées comme les phényltriméthicones, les phényldiméthicones, les phényltriméthylsiloxydiphényl-siloxanes, les diphényl-diméthicones, les diphénylméthyldiphényl trisiloxanes, les 2-phényléthyltriméthyl-siloxysilicates, et les polyméthylphénylsiloxanes ;
- leurs mélanges.

On entend par « huile hydrocarbonée » dans la liste des huiles citées ci-dessus, toute huile comportant majoritairement des atomes de carbone et d'hydrogène, et éventuellement des groupements ester, éther, fluoré, acide carboxylique et/ou alcool.

Les autres corps gras pouvant être présents dans la phase huileuse sont par exemple les acides gras comportant de 8 à 30 atomes de carbone, comme l'acide stéarique, l'acide laurique, l'acide palmitique et l'acide oléique ; les cires comme la lanoline, la cire d'abeille, la cire de Carnauba ou de Candellila, les cires de paraffine, de lignite ou les cires microcristallines, la cérésine ou l'ozokérite, les cires synthétiques comme les cires de polyéthylène, les cires de Fischer-Tropsch ; les résines de silicone telles que la trifluorométhyl-C1-4-alkyldiméthicone et la trifluoropropyldiméthicone ; et les élastomères de silicone comme les produits commercialisés sous les dénominations « KSG » par la société Shin-Etsu, sous les dénominations « Trefil », « BY29 » ou « EPSX » par la société Dow Corning ou sous les dénominations « Gransil » par la société Grant Industries.

Ces corps gras peuvent être choisis de manière variée par l'homme du métier afin de préparer une composition ayant les propriétés, par exemple de consistance ou de texture, souhaitées.

Les émulsions contiennent généralement au moins un émulsionnant choisi parmi les émulsionnants amphotères, anioniques, cationiques ou non ioniques, utilisés seuls ou en mélange, et éventuellement un co-émulsionnant. Les émulsionnants sont choisis de manière appropriée suivant l'émulsion à obtenir (E/H ou H/E). L'émulsionnant et le co-émulsionnant sont généralement présents dans la composition, en une proportion allant de 0,3 à 30 % en poids, et de préférence de 0,5 à 20 % en poids par rapport au poids total de la composition.

Pour les émulsions E/H, on peut citer par exemple comme émulsionnants les diméthicone copolyols tels que le mélange de cyclométhicone et de diméthicone copolyol, vendu sous la dénomination « DC 5225 C » par la société Dow Corning, et les alkyl-diméthicone copolyols tels que le Laurylméthicone copolyol vendu sous la dénomination « Dow Corning 5200 Formulation Aid » par la société Dow Corning et le cétyl diméthicone copolyol vendu sous la dénomination « Abil EM 90® » par la société Goldschmidt. On peut aussi utiliser comme tensioactif d'émulsions E/H, un organopolysiloxane solide élastomère réticulé comportant au moins un groupement oxyalkyléné, tel que ceux obtenus selon le mode opératoire des exemples 3, 4 et 8 du document US-A-5,412,004 et des exemples du document US-A-5,811,487, notamment le produit de l'exemple 3 (exemple de synthèse) du brevet US-A-5,412,004 et tel que celui commercialisé sous la référence KSG 21 par la société Shin Etsu.

Pour les émulsions H/E, on peut citer par exemple comme émulsionnants, les émulsionnants non ioniques tels que les esters d'acides gras et de glycérol oxyalkylénés (plus particulièrement polyoxyéthylénés) ; les esters d'acides gras et de sorbitan oxyalkylénés ; les esters d'acides gras oxyalkylénés (oxyéthylénés et/ou oxypropylénés) ; les éthers d'alcools gras oxyalkylénés (oxyéthylénés et/ou oxypropylénés) ; les esters de sucres comme le stéarate de sucrose ; et leurs mélanges tels que le mélange de stéarate de glycéryle et de stéarate de PEG-40.

Ces compositions peuvent être également des émulsions H/E stabilisées par des particules comme par exemple des particules polymériques décrites dans le brevet FR2760641, des polymères amphiphiles réticulés ou non tels que décrits dans les demandes : FR2853543 et FR2819175.

De façon connue, la composition cosmétique peut contenir également des adjuvants habituels dans le domaine cosmétique, tels que les gélifiants hydrophiles ou lipophiles, les additifs hydrophiles ou lipophiles, les conservateurs, les antioxydants, les solvants, les parfums, les charges, les absorbeurs d'odeurs et les matières colorantes. Les quantités de ces différents adjuvants sont celles classiquement utilisées dans le domaine cosmétique, et par exemple varient d'environ 0,01 % à 10 % du poids total de la composition. Ces adjuvants, selon leur nature, peuvent être introduits dans la phase grasse, dans la phase aqueuse et/ou dans les sphérules lipidiques.

Comme solvants utilisables dans l'invention, on peut citer les alcools inférieurs notamment l'éthanol, l'isopropanol, le dipropylène glycol, le butylène glycol, et le propylène glycol.

Comme gélifiants hydrophiles utilisables dans l'invention, on peut citer à titre d'exemples non limitatifs, les polymères carboxyvinyliques (carbomer®), les copolymères acryliques tels que les copolymères d'acrylates/alkylacrylates, les polyacrylamides, les polysaccharides tels que l'hydroxypropylcellulose, les gommes naturelles et les argiles, et, comme gélifiants lipophiles, on peut citer les argiles modifiées comme les bentones, les sels métalliques d'acides gras comme les stéarates d'aluminium, la silice hydrophobe, l'éthylcellulose et le polyéthylène.

Lorsque le monosaccharide est administré par voie orale, la composition le contenant peut être avantageusement sous la forme d'une gélule, d'un comprimé, ou de pilules. Lorsque le monosaccharide est administré par injection cutanée, la composition le contenant peut être en particulier sous la forme d'une solution stérile.

Les compositions de l'invention peuvent contenir d'autres actifs hydrophiles ou lipophiles. Ces actifs sont notamment choisis parmi les agents anti-oxydants, les agents dermo-relaxants ou dermodécontractants, des agents anti-âge, les agents anti-glycation, les agents stimulants la synthèse de macromolécules dermiques ou épidermiques et/ou empêchant leur dégradation, les agents stimulants la prolifération des fibroblastes ou des kératinocytes et/ou la différenciation des kératinocytes, les agents favorisant la maturation de l'enveloppe cornée, les inhibiteurs de NO synthase et les agents stimulant le métabolisme énergétique des cellules.

Agents anti-âge :

Parmi les actifs connus pour lutter contre les signes du vieillissement, notamment cutané, on peut citer notamment :

la vitamine B3, le coenzyme Q10 (ou ubiquinone), la vitamine B9, la vitamine E, les dérivés de la vitamine E, tels que le dérivé phosphaté comme par exemple le TPNA® commercialisé par la société Showa Denko, le resvératrol ou ses dérivés, comme par exemple le resveratrate® commercialisé par la société Estée Lauder, le rétinol ou ses dérivés, et leur mélange.

Agents anti-glycation:

Par « agent anti-glycation », on entend un composé prévenant et/ou diminuant la glycation des protéines de la peau, en particulier des protéines du derme, telles que le collagène.

Comme agents anti-glycation, on peut citer notamment les extraits végétaux de la famille des *Ericaceae,* tels qu'un extrait de myrtille (*Vaccinium angusfifollium, Vaccinium myrtillus),* par exemple celui vendu sous la dénomination « BLUEBERRY HERBASOL EXTRACT PG » par la société COSMETOCHEM, l'ergothionéine et ses dérivés, les hydroxystilbènes et leurs dérivés, tels que le resvératrol et le 3,3', 5,5'-tétrahydroxystilbène (ces agents anti-glycation sont décrits dans les demandes FR 2 802 425, FR 2 810 548, FR 2 796 278 et FR 2 802 420, respectivement), les dihydroxystilbènes et leurs dérivés, les polypeptides d'arginine et de lysine tels que celui vendu sous la dénomination « AMADORINE® » par la société SOLABIA, le chlorhydrate de carcinine (commercialisé par Exsymol sous la dénomination « ALISTIN®») , un extrait *d'Hélianthus annuus* comme l'Antiglyskin® de SILAB, les extraits de vin tel que l'extrait de vin blanc en poudre sur support maltodextrine vendu sous la dénomination « Vin blanc déshydraté 2F » par la société Givaudan, l'acide thioctique (ou acide alpha lipoïque), un mélange d'extrait de busserole et de glycogène marin comme l'Aglycal LS 8777® des Laboratoires Sériobiologiques, un extrait de thé noir comme le Kombuchka® de Sederma et leurs mélanges.

Comme agents anti-glycation préférés, on citera les extraits de myrtille *(Vaccinium myrtillus)* et l'extrait de thé noir.

Agents stimulant la synthèse de macromolécules dermiques et/ou épidermiques et/ou empêchant leur dégradation :

Parmi les actifs stimulant les macromolécules du derme ou empêchant leur dégradation, on peut citer ceux qui agissent :

- soit sur la synthèse du collagène, tels que les extraits de Centella asiatica, les asiaticosides et dérivés ; les peptides de synthèse tels que la iamin, le biopeptide CL ou palmitoyloligopeptide commercialisé par la société SEDERMA ; les peptides extraits de végétaux, tels que l'hydrolysat de soja commercialisé par la société COLETICA sous la dénomination commerciale Phytokine® ; les peptides de riz tel que le Nutripeptide® de SILAB, le méthylsilanol mannuronate tel que l'Algisium C® commercialisé par Exsymol ; les hormones végétales telles que les auxines et les lignanes ; l'acide folique ; et un extrait de Medicago sativa (alfafa) tel que celui commercialisé par SILAB sous la dénomination Vitanol®; un extrait peptidique de noisette tel que celui commercialisé par la société Solabia sous la dénomination Nuteline C®; et l'arginine.

- soit sur l'inhibition de la dégradation du collagène, en particulier des agents agissant sur l'inhibition des métalloprotéinases (MMP) telles que plus particulièrement les MMP 1, 2, 3, 9 . On peut citer : les rétinoïdes et dérivés, les extraits de medicago sativa tels que le Vitanol® de Silab, un extrait d'aphanizomenon flos-aquae (cyanophycée) commercialisé sous la dénomination Lanablue® par Atrium Biotechnologies, les oligopeptides et les lipopeptides, les lipoaminoacides, l'extrait de malt commercialisé par la société COLETICA sous la dénomination commerciale Collalift® ; les extraits de myrtille ou de romarin ; le lycopène ; les isoflavones, leurs dérivés ou les extraits végétaux en contenant, en particulier les extraits de soja (commercialisé par exemple par la société ICHIMARU PHARCOS sous la dénomination commerciale Flavostérone SB®), de trèfle rouge, de lin, de kakkon; un extrait de litchi ; la dipalmitoyl hydroxyproline commercialisée par Seppic sous le nom SEPILIFT DPHP® : Baccharis genistelloide ou Baccharine commercialisé par SILAB, un extrait de moringa tel que l'Arganyl LS 9781® de Cognis ; l'extrait de sauge décrit dans la demande FR-A-2812544 de la famille des labiées (*salvia officinalis* de la société Flacksmann), l'extrait de Rhododendron, le blueberry extract, un extrait de *vaccinium myrtillus* tel que ceux décrits dans la demande FR-A-2814950.

- soit sur la synthèse de molécules appartenant à la famille des élastines (élastine et fibrilline), tels que : le rétinol et dérivés en particulier le palmitate de rétinol ; l'extrait de *Saccharomyces Cerivisiae* commercialisé par la société LSN sous la dénomination commerciale Cytovitin® ; et l'extrait d'algue *Macrocystis pyrifera* commercialisé par la société SECMA sous la dénomination commerciale Kelpadelie® ; un extrait peptidique de noisette tel que celui commercialisé par la société Solabia sous la dénomination Nuteline C®.

- soit sur l'inhibition de la dégradation de l'élastine tels que l'extrait peptidique de graines de *Pisum sativum* commercialisé par la société LSN sous la dénomination commerciale Parelastyl® ; les héparinoïdes ; et les composés N-acylaminoamides décrits dans la demande WO 01/94381 tels que l'acide {2-[acétyl-(3-trifluorométhyl-phényl)-amino]-3-méthyl-butyrylamino} acétique, autrement nommé N-[N-acétyl, N'-(3-trifluorométhyl)phényl valyl]glycine ou N-acétyl-N-[3-(trifluorométhyl)phényl]valyl-glycine ou acétyl trifluorométhyl phényl valylglycine, ou un ester de celui-ci avec un alcool en $C_1$-$C_6$. ; un extrait de peptides de riz tel que la Colhibin® de Pentapharm, ou un extrait de Phyllanthus emblica tel que l'Emblica® de Rona.

- soit sur la synthèse des glycosaminoglycannes, tels que le produit de fermentation du lait par lactobacillus vulgaris, commercialisé par la société BROOKS sous la dénomination commerciale Biomin yogourth® ; l'extrait d'algue brune Padina pavonica commercialisé par la société ALBAN MÜLLER sous la dénomination commerciale HSP3® ; l'extrait de *Saccharomyces cerevisiae* disponible notamment auprès de la société SILAB sous la dénomination commerciale Firmalift® ou auprès de la société LSN sous la dénomination commerciale Cytovitin® ; un extrait de *Laminaria ochroleuca* tel que la Laminaïne® de Secma ; l'essence de Mamaku de Lucas Meyer, un extrait de cresson (Odraline® de Silab).

- soit sur la synthèse de la fibronectine, tels que l'extrait de zooplancton Salina commercialisé par la société SEPORGA sous la dénomination commerciale GP4G® ; l'extrait de levure disponible notamment auprès de la société ALBAN MÜLLER sous la dénomination commerciale Drieline® ; et le palmitoyl pentapeptide commercialisé par la société SEDERMA sous la dénomination commerciale Matrixil®.

Parmi les actifs stimulant les macromolécules épidermiques, telles que la fillagrine et les kératines, on peut citer notamment l'extrait de lupin commercialisé par la société SILAB sous la dénomination commerciale Structurine® ; l'extrait de bourgeons de hêtre *Fagus sylvatica* commercialisé par la société GATTEFOSSE sous la dénomination commerciale Gatuline® RC; et l'extrait de zooplancton Salina commercialisé par la société SEPORGA sous la dénomination commerciale GP4G® ; Tripeptide de Cuivre de PROCYTE ; un extrait peptidique de *Voandzeia substerranea* tel que celui commercialisé par la société Laboratoires Sérobiologiques sous la dénomination commerciale Filladyn LS 9397®.

De préférence, on utilisera un actif stimulant la synthèse de macromolécules dermiques et/ou épidermiques et/ou empêchant leur dégradation choisi parmi les agents stimulant la synthèse des glycosaminoglycanes, les agents inhibant la dégradation de l'élastine, les agents stimulant la synthèse de la fibronectine, les agents stimulant la synthèse de macromolécules épidermiques, et leurs mélanges.

Encore plus préférentiellement, on utilisera un actif stimulant la synthèse des glycosaminoglycanes choisi parmi un extrait d'algue brune Padina pavonica, un extrait de *Saccharomyces cerevisiae*, un extrait de *Laminaria ochroleuca,* l'essence de Mamaku, un extrait de cresson et leurs mélanges.

Comme actifs préférés stimulant la synthèse de macromolécules dermiques et/ou épidermiques et/ou empêchant leur dégradation, on peut citer :

les peptides de synthèse tels que la iamin, le biopeptide CL ou palmitoyloligopeptide commercialisé par la société SEDERMA ; les peptides extraits de végétaux, tels que l'hydrolysat de soja commercialisé par la société COLETICA sous la dénomination commerciale Phytokine® ; les peptides de riz tel que le Nutripeptide® de SILAB, le méthylsilanol mannuronate tel que l'Algisium C® commercialisé par Exsymol ; l'acide folique ; un extrait de Medicago sativa (alfafa) tel que celui commercialisé par SILAB sous la dénomination Vitanol® ; un extrait peptidique de noisette tel que celui commercialisé par la société Solabia sous la dénomination Nuteline C® ; l'arginine ; un extrait d'aphanizomenon flos-aquae (cyanophycée) commercialisée sous la dénomination Lanablue® par Atrium Biotechnologies, l'extrait de malt commercialisé par la société COLETICA sous la dénomination commerciale Collalift® le lycopène ; un extrait de litchi ; un extrait de moringa tel que l'Arganyl LS 9781® de Cognis ; un extrait de *vaccinium myrtillus* tel que ceux décrits dans la demande FR-A-2814950 ; le rétinol et dérivés en particulier le palmitate de rétinol ; l'extrait de *Saccharomyces Cerivisiae* commercialisé par la société LSN sous la dénomination commerciale Cytovitin® ; un extrait peptidique de noisette tel que celui commercialisé par la société Solabia sous la dénomination Nuteline C®; l'acide {2-[acétyl-(3-trifluorométhyl-phényl)-amino]-3-méthyl-butyrylamino} acétique, autrement nommé N-[N-acétyl, N'-(3-trifluorométhyl)phényl valyl]glycine ou N-acétyl-N-[3-(trifluorométhyl)phényl]valyl-glycine ou acétyl trifluorométhyl phényl valylglycine, ou un ester de celui-ci avec un alcool en $C_1$-$C_6$. ; un extrait de peptides de riz tel que la Colhibin® de Pentapharm, ou un extrait de Phyllanthus emblica tel que l'Emblica® de Rona ; l'extrait d'algue brune Padina pavonica commercialisé par la société ALBAN MÜLLER sous la dénomination commerciale HSP3® ; l'extrait de *Saccharomyces cerevisiae* disponible notamment auprès de la société SILAB sous la dénomination commerciale Firmalift® ou auprès de la société LSN sous la dénomination commerciale Cytovitin®· ; un extrait de *Laminaria ochroleuca* tel que la Laminaïne® de Secma ; l'essence de Mamaku de Lucas Meyer, l'extrait de lupin commercialisé par la société SILAB sous la dénomination commerciale Structurine® ; l'extrait de bourgeons de hêtre *Fagus sylvatica* commercialisé par la société GATTEFOSSE sous la dénomination commerciale Gatuline® RC.

Agents stimulant la prolifération des fibroblastes ou des kératinocytes et/ou la différenciation des kératinocytes

Les agents stimulant la prolifération des fibroblastes utilisables dans la composition selon l'invention peuvent par exemple être choisis parmi les protéines ou polypeptides végétaux, extraits notamment du soja (par exemple un extrait de soja commercialisé par la société LSN sous la dénomination Eleseryl SH-VEG 8® ou commercialisé par la société SILAB sous la dénomination commerciale Raffermine®) ; un extrait de protéines hydrolysées de soja tel que le RIDULISSE® de SILAB ; et les hormones végétales telles que les giberrellines et les cytokinines ; un extrait peptidique de noisette tel que celui commercialisé par la société Solabia sous la dénomination Nuteline C®.

De préférence on utilisera un agent favorisant la prolifération et/ou la différenciation des kératinocytes.

Les agents stimulant la prolifération des kératinocytes, utilisables dans la composition selon l'invention, comprennent notamment le phloroglucinol, l'extrait de feuille d'hydrangea macrophylla comme l'Amacha liquid E® d'Ichimaru Pharcos, un extrait de levure tel que le Stimoderm® de CLR ; l'extrait de *Larrea divaricata* tel que le Capislow® de Sederma, les mélanges d'extraits de papaye, de feuilles d'olivier et de citron tel que la Xyléine® de Vincience, l'extrait de feuille d'hydrangea macrophylla comme l'Amacha liquid E® d'Ichimaru Pharcos, le rétinol et ses esters dont le palmitate de rétinyle, le phloroglucinol, les extraits de tourteaux de noix commercialisés par Gattefosse et les extraits de *solanum tuberosum* tel que le Dermolectine® commercialisé par Sederma.

Parmi les agents stimulant la différenciation des kératinocytes comprennent par exemple les minéraux tels que le calcium; un extrait peptidique de lupin tel que celui commercialisé par la société SILAB sous la dénomination commerciale Structurine® ; le beta-sitosteryl sulfate de sodium tel que celui commercialisé par la société SEPORGA sous la dénomination commerciale Phytocohésine® ; et un extrait hydrosoluble de maïs tel que celui commercialisé par la société SOLABIA sous la dénomination commerciale Phytovityl® ; un extrait peptidique de *Voandzeia substerranea* tel que celui commercialisé par la société Laboratoires Sérobiologiques sous la dénomination commerciale Filladyn LS 9397® ; et les lignanes tels que le sécoisolaricirésinol, le rétinol et ses esters dont le palmitate de rétinyle.

Comme agents stimulant la prolifération et/ou la différenciation des kératinocytes, on peut citer encore les oestrogènes, tels que l'oestradiol et homologues ; ou les cytokines.

Comme actifs stimulant la prolifération des fibroblastes ou des kératinocytes et/ou la différenciation des kératinocytes préférés, on citera des protéines ou polypeptides végétaux, extraits notamment du soja (par exemple un extrait de soja commercialisé par la société LSN sous la dénomination Eleseryl SH-VEG 8® ou commercialisé par la société SILAB sous la dénomination commerciale Raffermine®) ; un extrait de protéines hydrolysées de soja tel que le RIDULISSE® de SILAB ; un extrait peptidique de noisette tel que celui commercialisé par la société Solabia sous la dénomination Nuteline C®; le phloroglucinol, un extrait de levure tel que le Stimoderm® de CLR ; un extrait peptidique de lupin tel que celui commercialisé par la société SILAB sous la dénomination commerciale Structurine®; un extrait hydrosoluble de maïs tel que celui commercialisé par la société SOLABIA sous la dénomination commerciale Phytovityl® ; un extrait peptidique de *Voandzeia substerranea* tel que celui commercialisé par la société Laboratoires Sérobiologiques sous la dénomination commerciale Filladyn LS 9397® ; ou encore le rétinol et ses esters dont le palmitate de rétinyle.

Agents favorisant la maturation de l'enveloppe cornée

On pourra utiliser dans les compositions de l'invention des agents qui interviennent sur la maturation de l'enveloppe cornée qui s'altère avec l'âge et induit une diminution de l'activité des transglutaminases. On peut citer par exemple l'urée et ses dérivés et en particulier l'Hydrovance® de National Starch et les autres actifs mentionnés dans la demande L'OREAL FR2877220.

Inhibiteurs de NO-synthases

L'agent ayant une action d'inhibiteur de NO synthase peut être choisi parmi les OPC (oligomères procyanidoliques) ; les extraits de végétal de l'espèce *Vitis vinifera* notamment commercialisés par la société Euromed sous la dénomination Leucocyanidines de raisins extra, ou encore par la société Indena sous la dénomination Leucoselect®, ou enfin par la société Hansen sous la dénomination Extrait de marc de raisin ; les extraits de végétal de l'espèce *Olea europaea* de préférence obtenus à partir de feuilles d'olivier et notamment commercialisés par la société VINYALS sous forme d'extrait sec, ou par la société Biologia & Technologia sous la dénomination commerciale Eurol® BT ; les extraits d'un végétal de l'espèce *Gingko biloba* de préférence un extrait aqueux sec de ce végétal vendu par la société Beaufour sous le nom commercial Ginkgo biloba extrait standard et leurs mélanges.

Agents stimulant le métabolisme énergétique des cellules

L'actif stimulant le métabolisme énergétique des cellules peut par exemple être choisi parmi la biotine, un extrait de *Saccharomyces cerevisiae* tel que le Phosphovital® de Sederma, le mélange de sels de sodium, de manganèse, de zinc et de magnésium d'acide pyrrolidone carboxylique comme le Physiogenyl® de Solabia, un mélange de gluconate de zinc, de cuivre et de magnésium tel que le Sepitonic M3® de Seppic et leurs mélanges ; un beta-glucan issu de *Saccharomyces cerevisiae* tel que celui commercialisé par la société Mibelle AG Biochemistry ;

Le monosaccharide selon l'invention peut être appliqué sur la partie de la peau ou des phanères à traiter, en particulier sur le visage, le cou, les mains, les cheveux ou le cuir chevelu, de préférence de façon quotidienne ou pluriquotidienne. L'application sera par exemple renouvelée tous les jours pendant une période variable selon les effets souhaités, généralement de 3 à 6 semaines, mais pourra être prolongée ou poursuivie en continu.

Selon une alternative, le monosaccharide selon l'invention ou les compositions le contenant pourront être administrés par voie injectable en association ou non avec des produits de comblement. En effet, l'une des solutions retenues pour lutter contre les rides et/ou la perte de volume des tissus mous est l'utilisation de produits de comblement (ou "filler"). Ce comblement peut être réalisé par l'utilisation de produits non résorbables, tels que des gels de polyacrylamide ou des particules de polyméthylméthacrylate (PMMA). Néanmoins, ces composés peuvent entraîner des réactions d'intolérance du type inflammation ou hypersensibilité.

On privilégie l'utilisation de composants résorbables, tels que les protéines, les graisses, le collagène ou l'acide hyaluronique. Mais ces composés sont dégradés assez rapidement dans l'organisme, ce qui réduit leur efficacité. Pour y remédier il faut donc procéder à une réticulation plus ou moins poussée de ces composants.

A ce jour, l'acide hyaluronique utilisé dans des formes pharmaceutiques ou des dispositifs médicaux se présente sous la forme d'un gel de hyaluronate de sodium.

Le monosaccharide selon l'invention ou les compositions les contenant pourront également être appliqués par mésothérapie.

La mésothérapie est une technique de traitement par injection intraépidermique et/ou intradermique et/ou sous-cutanée de produit(s) actif(s), comme par exemple des micro-nutriments, des vitamines, et/ou de l'acide hyaluronique. Les compositions sont administrées selon cette technique par injection sous forme de multiples gouttelettes de faible taille au niveau de l'épiderme, de la jonction dermo-épidermique et/ou du derme afin, notamment, de réaliser un nappage sous-cutané. La technique de mésothérapie est notamment décrite dans l'ouvrage « Traité de mésothérapie » de Jacques LE COZ, édition Masson, 2004.

La mésothérapie faite sur le visage est également appelée mésolift, ou également sous le terme anglosaxon de « mesoglow ».

Ainsi, un autre objet de la présente invention peut être un dispositif, en particulier un dispositif médical, comprenant au moins un monosaccharide tel que défini ci-dessus, le dispositif étant adapté à une injection intraépidermique et/ou intradermique et/ou sous-cutanée. Ledit monosaccharide est de préférence en association avec une solution stérile. Ledit dispositif peut comprendre au moins un autre composé, comme au moins un produit résorbable ou non, tel que ceux mentionnés ci-dessus, éventuellement réticulé.

Ledit dispositif peut être par exemple une seringue avec une aiguille ou encore un dispositif injecteur sans aiguille, tel que ceux utilisés dans la technique de soin connue sous le nom de mésothérapie. Un kit comprenant un dispositif peut être également envisagé, ledit kit comprenant un dispositif, en particulier une seringue ou un dispositif injecteur, et au moins un monosaccharide tel que défini ci-dessus. Ledit kit peut comprendre également une aiguille. Ledit dispositif peut se trouver prêt à l'emploi, c'est à dire pré-rempli, ou doit être rempli lors de l'utilisation. Dans ce dernier cas, une composition ou un autre dispositif (comme une ampoule) comprend ledit monosaccharide, éventuellement en association avec au moins un autre composé actif, comme au moins un produit résorbable ou non, tel que les produits de comblement mentionnés ci-dessus, éventuellement réticulé.

L'injection du monosaccharide selon l'invention peut être réalisé simultanément à, ou avant ou après, l'application sur la peau ou ses phanères d'une autre composition cosmétique ou pharmaceutique, de préférence dermatologique, comprenant, dans un support physiologiquement acceptable, au moins un autre actif, tel que cité ci-dessus.

**Légende des Figures**

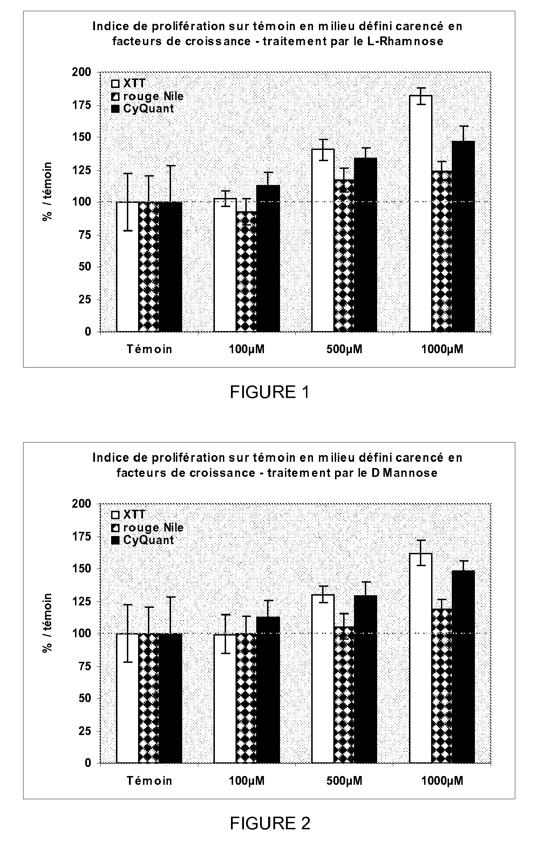

**Figure 1 :** Diagramme schématisant les résultats obtenus pour la prolifération des kératinocytes, en présence d'un témoin, en présence de différents marqueurs, en milieu carencé en facteurs de croissance, et avec ajout de différentes concentrations en L-Rhamnose reportées en abscisse. Les valeurs reportées en ordonnée correspondent aux pourcentages de cellules marquées mesurés par rapport au témoin.

**Figure 2 :** Diagramme schématisant les résultats obtenus pour la prolifération des kératinocytes, en présence d'un témoin, en présence de différents marqueurs, en milieu carencé en facteurs de croissance, et avec ajout de différentes concentrations en D-Mannose reportées en abscisse. Les valeurs reportées en ordonnée correspondent aux pourcentages de cellules marquées mesurés par rapport au témoin.

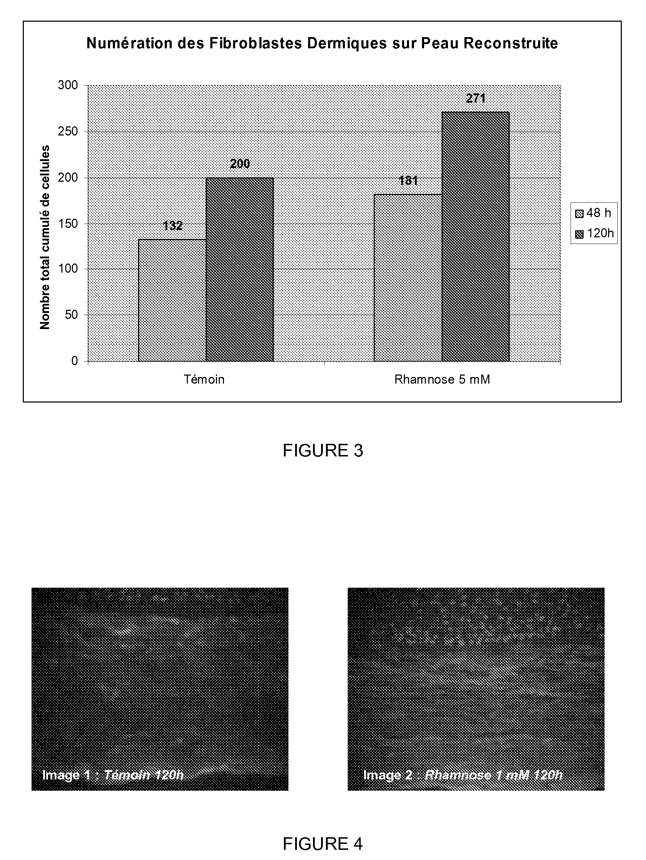

**Figure 3 :** Diagramme représentant le nombre de fibroblastes mesurés entre une peau reconstruite complète témoin non traité, à gauche, et une peau reconstruite complète traitée par 5 mM de rhamnose, à droite. Les fibroblastes sont comptés à différents stades du traitement. Ainsi, pour chaque type de peau la colonne de gauche correspond à la numérotation effectuée à 48 h et la colonne de droite correspond à la numérotation effectuée à 120 h de traitement.

**Figure 4 :** Photographies de coupe à congélation de peau reconstruite de $7\,\mu M$ d'épaisseur. Le niveau de fluorescence se matérialise par les tâches blanche du cliché en noir et blanc, il est proportionnel à la quantité de procollagène de type I. A gauche figure la peau témoin et à droite la peau traitée par 1 mM de rhamnose.

L'invention est illustrée plus en détail dans les exemples suivants qui sont présentés à titre illustratif et non limitatif du domaine de l'invention.

## Exemples

### Exemple 1 : Prolifération des kératinocytes

### Protocole

Les kératinocytes (lignée HaCat) sont cultivés dans deux conditions : milieu de culture défini complet (condition standard) et milieu de culture carencé en facteurs de croissance. Ce milieu carencé entraîne un retard maitrisé de la prolifération cellulaire. Dans ces conditions, il est alors possible de mesurer les effets de composés, capables de compenser la carence en facteurs de croissance du milieu de culture et donc de relancer la multiplication cellulaire et /ou de stimuler leur métabolisme.
La prolifération kératinocytaire est mesurée au moyen de trois marqueurs sur la même population cellulaire : le taux d'ADN qui est proportionnel au nombre de cellules (sonde Cyquant), le taux de lipides polaires constitutifs de membranes cellulaires (sonde Rouge Nil) et la respiration mitochondriale, qui réflète le métabolisme cellulaire général (sonde XTT).

### Résultats

Les résultats sont donnés dans les figures 1 et 2.
Les deux monosaccharides Rhamnose et Mannose démontrent leur capacité à activer la prolifération des kératinocytes, lorsque ceux-ci sont cultivés en milieu appauvri en facteurs de croissance, condition de culture qui retarde significativement leur croissance cellulaire.
Cette activation de la prolifération cellulaire par les deux composés se manifeste par un nombre de cellules plus important par rapport au témoin non traité.
Ce nombre augmenté de cellules se matérialise par un taux d'ADN (Cyquant), un taux de lipides polaires (signal Rouge Nil) et une respiration mitochondriale (signal XTT) significativement augmentés lorsque les monosaccharides sont évalués à 1 mM. A 500 $\mu M$, les deux molécules présentent déjà une efficacité. Les deux monosaccharides mannose et rhamnose exercent donc une influence sur la prolifération des kératinocytes. Ils activent la prolifération des kératinocytes cultivés en milieu appauvri en facteur de croissance, ce qui se manifeste par un nombre de cellules plus important par rapport au témoin non traité.
Le rhamnose et le mannose présentent donc une efficacité anti âge intéressante en venant booster le renouvellement épidermique et lutter contrer l'atrophie épidermique liée au vieillissement.

**Exemple 2 : Prolifération des fibroblastes**

**Protocole**

Le rhamnose a été étudié sur un modèle de peau reconstruite complète afin de mesurer son efficacité anti âge au niveau du compartiment dermique.

Brièvement, le modèle de peau reconstruite utilisé est celui décrit par Bell et al, (Bell E. et al, The reconstitution of living skin, J Invest Dermatol, 1983, Jul ;81) : il comporte un équivalent dermique sur lequel est reconstruit un épiderme pluristratifié ; l'équivalent dermique est fabriqué à partir de collagène acido soluble, de milieu de culture contenant du sérum et de fibroblastes humains normaux adultes. Après 5 jours de rétraction, cet équivalent est ensemencé avec des kératinocytes puis cultivé durant 6 jours en immersion et 7 jours à émersion afin d'obtenir un épiderme pluristratifié et différencié présentant une couche cornée.

La peau reconstruite est traitée par du rhamnose à 5 mM pendant 2 jours et 5 jours dans le milieu de culture ; à l'issue du traitement, les peaux reconstruites sont incluses en Tissue Tek en vue de coupes à congélation de 7$\mu$M d'épaisseur au cryostat. Les coupes réalisées sont ensuite marquées à l'iodure de propidium pour marquer l'ADN des noyaux des fibroblastes en vue de leur numération. 3 coupes à congélation sont réalisées aléatoirement sur chaque peau reconstruite ; sur chaque coupe, 2 champs microscopiques (objectif X25) sont analysés en microscopie à fluorescence et photographiés. La numération des fibroblastes dermiques est donc réalisée pour chaque peau reconstruite sur 6 images au total représentant les 6 champs microscopiques considérés. Le nombre de fibroblastes dermiques est comparé entre la peau témoin et celle traitée par le rhamnose aux deux temps de la cinétique.

**Résultats**

Les résultats sont donnés à la figure 3.

Il a été constaté que le rhamnose induit la stimulation de la croissance des fibroblastes dermiques de la peau reconstruite dès 48 heures de traitement, stimulation confirmée à 120 h de traitement, avec entre 30 à 35 % de cellules en plus (voir figure 3). A noter que cette stimulation s'accompagne d'une stimulation de la synthèse de procollagène 1 à 5 mM, ainsi qu'à 1 mM, ce qui peut également résulter du nombre accru des fibroblastes responsable de la sécrétion de cette protéine majeure de la matrice extracellulaire.

Ces deux effets complètent l'activité anti-âge du rhamnose déjà mesurée sur le compartiment épidermique, en venant stimuler la prolifération et le métabolisme du fibroblaste, cellule majeure du compartiment dermique.

**Exemple 3 : Synthèse de Procollagène 1**

Il a été procédé également sur d'autres séries de coupes à congélation à la détection classique par immunofluorescence indirecte du procollagène de type I au niveau du derme de la peau reconstruite (Anticorps anti procoll 1 *(MAB 1912 Millipore)* + conjugué couplé à FITC *(112-095-068 Jackson Immunoresearch)*). Afin de bien se repérer au sein de l'architecture cutanée lors de l'examen microscopique des coupes, les noyaux cellulaires des kératinocytes et des fibroblastes sont localisés grâce à leur marquage à l'iodure de propidium, comme décrit plus haut. 3 coupes à congélation sont réalisées aléatoirement sur chaque peau reconstruite et sur chaque coupe, 2 champs microscopiques (objectif X25) sont analysés en microscopie à fluorescence et photographiés. Les niveaux de fluorescence proportionnels à la quantité de procollagène de type I sont comparés entre la peau témoin et la peau traitée par le rhamnose.

Sur l'image 1, figure 4, correspondant à une coupe de la peau reconstruite témoin à 120h de culture, la présence du procollagène de type 1 synthétisé par les fibroblastes dermiques, se matérialise par la fluorescence verte située dans la partie inférieure de l'image. On devine sur la partie supérieure de l'image, la partie basale de l'épiderme, tissu très cellulaire, visualisable par les nombreux noyaux des kératinocytes. On visualise également dans le derme ; tissu beaucoup moins cellulaire, la distribution aléatoire des fibroblastes au sein de la matrice extracellulaire dermique.

Sur l'image 2, figure 4, correspondant par exemple, à une coupe de la peau reconstruite traitée par le Rhamnose à 1 mM pendant 120 heures, on constate une nette augmentation de la fluorescence verte comparativement à celle observée pour la peau témoin (image 1) ainsi qu'une distribution du signal fluorescent matérialisant bien l'aspect fibrillaire du procollagène de type I néosynthétisé. Cette augmentation de la fluorescence générale indique que le traitement par le rhamnose a fortement stimulé la synthèse du procollagène de type I par les fibroblastes.

Ces résultats montrent bien la capacité du rhamnose à stimuler le métabolisme du fibroblaste, métabolisme qui au cours du vieillissement, se déséquilibre plus vers la dégradation de la matrice extracellulaire que vers son renouvellement.

Le rhamnose en stimulant à la fois le métabolisme et la croissance des fibroblastes dermiques démontre bien son efficacité anti-âge sur le derme, efficacité complémentaire de celle mesurée vis-à-vis du compartiment épidermique.

**Exemple 4 : Compositions cosmétiques**

**- composition A**

| Crèmes régénération épidermiqe et dermique : émulsion huile dans eau | Comp. A |
|---|---|
| Ammonium Polyacryldimethyltauramide (Hostacerin AMPS de Clariant) | 1,00% |
| Cyclohexasiloxane | 5,0% |
| Glycerin | 1,70% |
| Stearyl alcohol | 0,30% |
| Glyceryl stearate / PEG-100 stearate | 0,70% |
| Dimyristyl tartrate / cetearyl alcohol / C12-15 pareth-7 / PPG-25 laureth-25 | 0,50% |
| Gomme de Xanthan | 0,20% |
| rhamnose | 5% |
| Conservateurs | 0,50% |
| Eau | qsp 100 |

**- composition B**

| Crèmes régénération épidermique : émulsion huile dans eau | Comp. B |
|---|---|
| Ammonium Polyacryldimethyltauramide (Hostacerin AMPS de Clariant) | 1,00% |
| Cyclohexasiloxane | 5,0% |
| Glycerin | 1,70% |
| Stearyl alcohol | 0,30% |
| Glyceryl stearate / PEG-100 stearate | 0,70% |
| Dimyristyl tartrate / cetearyl alcohol / C12-15 pareth-7 / PPG-25 laureth-25 | 0,50% |
| Gomme de Xanthan | 0,20% |
| mannose | 5% |
| Conservateurs | 0.50% |
| Eau | qsp 100 |

**- composition C**

**Crème de jour anti-âge pour le visage**

Phase A1 :-

| | |
|---|---|
| - Distéarate de sucrose commercialisé par la Société STEARINERIE DUBOIS | 1,75% |
| - Stéarate de sorbitane oxyéthyléné à 4 moles d'oxyde d'éthylène commercialisé par la Société ICI sous le nom "TWEEN 61" | 1,15% |
| - Acide stéarique | 0,75% |
| - Stearyl heptanoate | 4,00% |
| - Vaseline codex | 1,50% |
| - Huile d'avocat | 3,20% |

(suite)

| | |
|---|---|
| - Huile de jojoba | 3,00% |
| - Huile de silicone volatile | 2,70% |
| - Acétate de vitamine E | 1,00% |
| - Glycérides de Vitamine F | 3,00% |

<u>Phase A2</u> :

| | |
|---|---|
| Gomme de silicone commercialisée par DOW CORNING sous le nom "Q2-1403 Fluid" | 3,00 % |
| - Propylparaben | 0,2 % |
| - Parfum | 0,3% |

<u>Phase B</u> :-

| | |
|---|---|
| Glycérine | 3,00% |
| - Hydroxyproline | 1,00% |
| - D-panthenol | 1,00% |
| - Triéthanolamine | 0,35% |
| - Rhamnose | 3,00% |
| - Méthylparaben | 0,3 % |
| - Eau déminéralisée    q.s.p. | 100 % |

<u>Phase C</u>:

- Ammonium Polyacryldimethyltauramide (Hostacerin AMPS de Clariant)1%

**- <u>composition D</u>**

**Nanoémulsion anti-âge pour le visage et le corps**

Phase huileuse :

| | | |
|---|---|---|
| - Nikkol DGMS (Diglyceryl monostéarate) (société Nikko) | 4 | % |
| - Sel disodique de l'acide N-Stéaroyl L-glutamique | | |
| (Acylglutamate HS21 de la société AJINOMOTO) | 0,5 | % |
| - Huile de jojoba (P.M. d'environ 900) | 6 | % |
| - Huile de silicone volatile | 6 | % |
| - Stéarate d'isocétyle (P.M. = 508) | 6 | % |

Phase aqueuse :

| | | |
|---|---|---|
| - Glycérine | 5 | % |
| - Dipropylène glycol | 10 | % |
| - Eau | 55 | % |
| - Mannose | 4 | % |

On obtient une nanoémulsion transparente.

**Revendications**

**1.** Utilisation cosmétique d'au moins un monosaccharide pour diminuer ou prévenir l'amincissement du derme, la peau molle et/ou la peau amincie, dans laquelle le monosaccharide est choisi parmi le rhamnose, le mannose et leurs mélanges et dans laquelle le monosaccharide est sous forme de monomère.

2. Utilisation selon la revendication précédente, pour diminuer ou prévenir la dégradation des fibres de collagène.

3. Utilisation selon l'une quelconque des revendications précédentes, pour maintenir et/ou renforcer le derme, pour maintenir l'intégrité et/ou l'épaisseur des différentes couches de la peau, à savoir le derme et l'épiderme.

4. Utilisation selon l'une quelconque des revendications précédentes, pour augmenter la prolifération des fibroblastes.

5. Utilisation selon l'une quelconque des revendications précédentes, pour stimuler la synthèse de collagène.

6. Utilisation selon l'une quelconque des revendications précédentes, dans laquelle le monosaccharide est un mélange de rhamnose et de mannose.


**Patentansprüche**

1. Kosmetische Verwendung wenigstens eines Monosaccharids zur Verminderung oder Verhinderung des Dünnerwerdens der Dermis, der schlaffen Haut und/oder der dünner gewordenen Haut, wobei das Monosaccharid aus Rhamnose, Mannose und ihren Gemischen ausgewählt ist und wobei das Monosaccharid als Monomer vorliegt.

2. Verwendung gemäß vorhergehendem Anspruch zur Verminderung oder Verhinderung des Abbaus von Kollagenfasern.

3. Verwendung gemäß einem der vorhergehenden Ansprüche zur Erhaltung und/oder Verstärkung der Dermis, zur Erhaltung der Integrität und/oder der Dicke der verschiedenen Hautschichten, nämlich Dermis und Epidermis.

4. Verwendung gemäß einem der vorhergehenden Ansprüche zur Steigerung der Fibroblastenproliferation.

5. Verwendung gemäß einem der vorhergehenden Ansprüche zur Stimulierung der Kollagensynthese.

6. Verwendung gemäß einem der vorhergehenden Ansprüche, wobei das Monosaccharid ein Gemisch aus Rhamnose und Mannose ist.


**Claims**

1. A cosmetic use of at least one monosaccharide for reducing or preventing the thinning of dermis, flaccid skin and/or thinned skin, wherein the monosaccharide is chosen from rhamnose, mannose, and mixtures thereof, and wherein the monosaccharide is a monomer.

2. Use according to the previous claim, for reducing or preventing degradation of collagen fibres.

3. Use according to anyone of the preceding claims, for maintaining and/or reinforcing the dermis, for maintaining the integrity and/or thickness of the various layers of the skin, namely dermis and epidermis.

4. Use according to anyone of the preceding claims, for increasing fibroblasts proliferation.

5. Use according to anyone of the preceding claims, for increasing the synthesis of collagens.

6. Use according to anyone of the preceding claims, wherein the monosaccharide is a mixture of rhamnose and mannose.

FIGURE 1

FIGURE 2

## Numération des Fibroblastes Dermiques sur Peau Reconstruite

FIGURE 3

FIGURE 4

## RÉFÉRENCES CITÉES DANS LA DESCRIPTION

**Documents brevets cités dans la description**

- WO 2007128939 A
- FR 2900572
- US 20070025933 A
- WO 2005063194 A
- JP 2295912 A
- US 5412004 A
- US 5811487 A
- FR 2760641
- FR 2853543
- FR 2819175
- FR 2802425
- FR 2810548
- FR 2796278
- FR 2802420
- FR 2812544 A
- FR 2814950 A
- WO 0194381 A
- FR 2877220

**Littérature non-brevet citée dans la description**

- **JACQUES LE COZ.** Traité de mésothérapie. 2004
- **BELL E. et al.** The reconstitution of living skin. *J Invest Dermatol,* Juillet 1983, 81